Europäisches Patentamt

European Patent Office

Office européen des brevets

(11) Publication number: **0 330 123**
**A2**

## EUROPEAN PATENT APPLICATION

(21) Application number: 89102919.1

(22) Date of filing: 20.02.89

(51) Int. Cl.⁴: **G05D 9/12 , G01F 23/28 , C07C 2/54**

(30) Priority: 22.02.88 US 158462

(43) Date of publication of application:
30.08.89 Bulletin 89/35

(84) Designated Contracting States:
AT BE CH DE ES FR GB GR IT LI LU NL SE

(71) Applicant: **PHILLIPS PETROLEUM COMPANY
5th and Keeler
Bartlesville Oklahoma 74004(US)**

(72) Inventor: **Morgan, John Alan
1208 SE Harris
Bartlesville Oklahoma 74006(US)**
Inventor: **Hachmuth, Henry Karl
Rt. 3 Box 72 60
Bartlesville Oklahoma 74003(US)**

(74) Representative: **Geissler, Bernhard, Dr. jur.,
Dipl.-Phys. Patent- und Rechtsanwälte et al
Bardehle-Pagenberg-Dost-
Altenburg-Frohwitter-Geissler & Partner
Postfach 86 06 20
D-8000 München 86(DE)**

(54) **Utilization of a nuclear-type interface level detector/transmitter.**

(57) An apparatus and method are provided for measuring and/or controlling fluid interface levels occurring within a vessel, wherein the fluid interface levels are measured and/or controlled by the utilization of a nuclear level measurment device comprising a radiation source and a radiation detector. This nuclear measurment device is externally positioned on a generally vertical standpipe which is in fluid communication with the vessel containing the fluids whose interface level is to be measured and/or controlled. The vertical standpipe is externally positioned, in relationship to the vessel, such that the fluid interface levels within the standpipe correspond with the same levels to those within the vessel.

FIG. 1

# UTILIZATION OF A NUCLEAR-TYPE INTERFACE LEVEL DETECTOR/TRANSMITTER

## Field of the Invention

This invention relates broadly to a method and apparatus for measuring interface levels. In one aspect, this invention relates to a method and apparatus for measuring interface levels and a control system for maintaining a desired interface level within a vessel and/or pipe. Particularly, this invention relates to a method and apparatus for measuring liquid/vapor interface levels and/or liquid/liquid interface levels, contained in a common tank, vessel or pipe, for the purpose of controlling the location of such levels.

## Background of the Invention

In many commercial applications, it is often critical to measure and/or control fluid interface levels occuring between two fluids, wherein one is insoluble in the other and wherein one has a specific gravity greater than the other. Therses fluid interface levels can result between a vapor and a liquid or between two liquids. One example of a commercial process, wherein it is desirable to measure and/or control fluid interface levels, is in hydrocarbon refining processes.

Specifically, in the alkylation of hydrocarbons using a hydrofluoric acid, it is often critical that the location of liquid/vapor interface levels (e.g., a liquid hydrocarbon phase and a vapor phase) and liquid/liquid interface levels (e.g., liquid hydrofluoric acid phase and a liquid hydrocarbon phase) be measured and controlled for safety and economic reasons. One reason for this criticality is the need to insure that no hydrocarbon vapor and/or hydrofluoric acid is removed from the particular vessel when attempting to withdraw therefrom a liquid hydrocarbon product. Therefore, creating an interface level measurement system, suitable for process indication and control in HF Alkylation units, and which is not adversely affected by the corrosive tendencies of hydrofluoric acid, would be a significant advancement in the art. However, the measurement of such interface levels, especially when one of the materials is as highly corrosive as hydrofluoric acid, has been a problem of long standing.

While many methods currently exist which measure and control the interface levels occurring in hydrofluoric acid alkylation (henceforth, HF Alkylation) units, they are most temporary solutions to a continual problem. For example, one such apparatus measures interface levels by using differential pressure instruments. This method, wherein the sensing device actually contacts the hydrofluoric acid, has proven to be unreliable because of the attack on the thin diaphragms of the differential pressure instrument or the seal diaphragms of the same by the extremely corrosive tendencies of the hydrofluoric acid.

Another such apparatus to measure interface levels in HF Alkylation units incorporates a float or displacer. Since the float or displacer must again physically contact the hydrofluoric acid, its use is also unsatisfactory because the hydrofluoric acid will corrode the float and render the linkage inoperative.

Attempts have also been made to use a sight glass to measure interface levels in HF Alkylation units. This method has also proven to be ineffective due to the fact that the hydrofluoric acid attacks glass. In fact, hydrofluoric acid will attack almost any transparent medium of which a gauge glass could be commercially constructed.

The use of trycocks to measure interface levels has proven to be reliable. However, their use has associated therewith the inherent disadvantage of releasing quantities of liquid and vapor hydrocarbons as well as the highly corrosive hydrofluoric acid into the surrounding atmosphere. Another inherent disadvantage is that this method of measuring interface levels is not a continuous measurement process but an incremental one.

Due to the extremely corrosive tendencies of hydrofluoric acid and the potential safety hazards inherently associated therewith, attempts have been made to measure the interface levels occurring therein by utilizing detectors which need not contact the material to be measured. One such attempt employs the use of a radiation-type interface level measurement device having a point source of radiation. This point source of radiation is mounted on the external wall of the vessel which contains the liquid whose level is to be measured. The radiation from this point source is emitted as a fan-shaped beam. On the external wall, directly opposite the point source of radiation, a radiation detector cell is mounted. Since the absorption of radiation by any material is a function of the mass of the material contained between the radiation source and the radiation detector, as the interface levels change, various amounts of radiation are detected, thus affecting a change in the radiation detector's output. When this change occurs, minute electrical currents are generated which, when processed and amplified, can be used for level indication and/or process control purposes.

While the use of a point source of radiation adjacent an external wall of the vessel, containing the fluids whose interface level is to be measured, provides a non-contact measurement system which circumvents the problems caused by contact with corrosive fluids, this point source radiation-type measurement device is plagued by many problems.

The first such problem arises when attempting to calibrate this particular radiation-type interface level measurement device. Specifically, to properly calibrate this device, it is necessary to remove the vessel from commission. This generally entails a temporary shutdown of the HF Alkylation unit.

After the vessel has been removed from commission, it is necessary to fill the vessel with the fluid to be measured while noting the relationship between the fluid levels and the output of the radiation detector. Since the vessels used in HF Alkylation units can be as large as 20 feet tall and 20 feet in diameter, the cost of such an endeavor could be staggering.

Another problem resulting from attempting to calibrate the radiation-type interface level measuring device, as disclosed above, stems from the vessel's construction and configuration. Specifically, due to economic feasibility, vessels used in HF Alkylation units are often constructed from low carbon steel. Although this material exhibits some resistance to hydrofluoric acid, it inevitably succumbs to the corrosive tendencies thereof by reacting therewith and forming layers of scale which, with time, break off. In view of this continual scale forming and breaking off process, the walls of the vessels used in HF Alkylation units must be thick enough to compensate for this corrosion. Therefore, to compensate for the thickness of the walls and the diameter of the vessels used in HF Alkylation units, a fairly strong radiation source is needed. Since the strength of a radiation source is directly proportional to its cost and safety hazards associated therewith, the feasibility of utilizing such a radiation-type level measuring device is questionable.

Another problem of using a point source radiation-type measurement device, as described above, stems from the continual process of scale forming on the internal walls of the vessel and, inevitably, breaking off. As indicated above, the absorption of radiation by any material is a function of the material contained between the radiation source and the radiation detector. Therefore, if a point source of radiation is used as described above, the calibration of this level detection device would be invalidated if a piece of scale should suddenly fall from a location directly adjacent to the point source of radiation. If this occurs, the radiation detector would indicate that a change has occurred within the vessel even if the interface level has not changed.

Yet another problem resulting from attaching a radiation-type level detection device to the external walls of a vessel used in an HF Alkylation unit, pertains to the external and/or internal configuration of the vessel. Specifically, vessels used in HF Alkylation units have many different shapes, sizes, and thicknesses depending upon their desired function and the material which they are to contain. Moreover, many of these vessels also have internal baffles, trays, pipes, etc. located therein upon which scale can form and break off, as described above. Since the calibration of a radiation-type level detecting device must take all of the above factors into consideration, selecting the appropriate radiation-type detection device is often done on a case by case basis. This individualized analysis adds to the cost and detracts from the desirability of using radiation-type level detecting devices in conjunction with HF Alkylation units.

Accordingly, it is an object of this invention to provide an improved apparatus and method for measuring and/or controlling interface levels occurring in HF Alkylation units which circumvent the problems associated with the corrosive tendencies of the hydrofluoric acid and the scale forming problems often associated therewith.

Other objects, aspects and advantages of the invention will be apparent to those skilled in the art upon reading the specification in view of the accompanying figures and appended claims.

## Summary of the Invention

In accordance with the present invention, an apparatus and method are provided for measuring and/or controlling fluid interface levels occurring within a vessel, wherein the fluid interface levels are measured and/or controlled by the utilization of a nuclear level measurement device comprising a radiation source and a radiation detector. This nuclear measurment device is externally positioned on a generally vertical standpipe which is in fluid communication with the vessel containing the fluids whose interface level is to be measured and/or controlled. The vertical standpipe is externally positioned, in relationship to the vessel, such that the fluid interface levels within the standpipe correspond with the same levels to those within the vessel.

## Brief Description of the Drawings

A more complete appreciation of the invention disclosed herein and many of the attendant advantages thereof will be readily obtained as the same becomes better understood by reference to the following detailed description when considered in conjunction with the accompanying figures forming a part hereof, wherin like reference characters denote like parts in the various views.

FIGURE 1 is a schematic representation, partially in section, of an HF Alkylation unit having the measurement and control system of this invention incorporated therein.

FIGURE 2 is a cross-sectional view of a nuclear level measurement device employed to measure and control the fluid interface levels in the hydrofluoric acid settler illustrated in FIGURE 1.

## Detailed Description

The inventive process disclosed herein pertains to the measurement and/or control of fluid interface levels within vessels in a manner which does not contact the fluids whose interface level is to be measured and/or controlled. This measurement and/or control process is accomplished by utilizing a nuclear level measurement device. The nuclear measurement device of this invention comprises a radiation source and a radiation detector.

In accordance with this invention, the nuclear level measurement device is externally positioned onto a generally vertical standpipe which is in fluid communication with a vessel, such that the fluid interface levels within the standpipe correspond to at the same interface levels within the vessel. The radiation source of the nuclear level measurement device is adjacent an external wall of the standpipe while the radiation detector is adjacent the external wall on the opposite side thereof.

As stated earlier, the absorption of radiation by any material is a function of the mass of the material contained between the radiation source and the radiation detector. Therefore, as the fluid interface levels within the vessel and standpipe change, varying amounts of radiation are detected, thus, causing a change in the radiation detector's output. This change generates a signal which can be used for level indication and/or process control purposes.

For purposes of demonstrating the attendant advantages of this invention, it will be described in relation to its application within an HF Alkylation unit. It should be noted, however, that while this invention is disclosed in relation to this specific application, it will be seen that the application of this invention is not limited thereto. Specifically, this invention is applicable to any such process in which a non-contracting fluid interface level measurement and/or control system is desirable.

The lines designated as signal lines in the attached drawings are electrical, pneumatic or optical in the preferred embodiments of this invention. Generally, the signals provided from a nuclear level measurment device as incorporated in this invention are electrical in form. However, various transducing means may be used to convert parameters which characterize the process to a variety of forms or formats. For example, the control elements of the novel system can be implemented using electrical analog, digital electronic, pneumatic, hydraulic, mechanical, optical or other such similar types of equipment or any combination of one or more types of such equipment. While the presently preferred embodiment of this invention utilizes a combination of pneumatic final control elements in conjunction with electrical analog signal handling and translation apparatus, the method and apparatus of this invention can be implemented using a wide variety of specific equipment available to and understood by those skilled in the process control art. Likewise, the format of the various signals can be modified substantially in order to accommodate signal format requirements of the particular installation, safety factors, physical characteristics of the measuring or controlling instrument and other similar factors.

In accordance with a preferred embodiment of this invention, the nuclear level measurement device, used to measure and/or control fluid interface levels contained within vessels of an HF Alkylation unit, comprises: (1) a strip-type radiation source and (2) a radiation detector. In view of the possibility of scale forming and breaking off the internal walls of the standpipe implemented in accordance with this invention, a strip-type radiation source is preferred over a point-type radiation source. Specifically, since the gamma rays from a strip-type radiation source are emitted over the entire span of the strip, as opposed to being emitted from a single point, a strip-type radiation souce is less affected by scale formation and removal.

The radiation source suitable for use when practicing the embodiments of this invention (e.g., cesium-137 or cobalt-60) emits a collimated beam of gamma ray energy through the fluid-containing standpipe which is in fluid communication with the vessel. The gamma rays are absorbed in proportion to the fluid interface level within the standpipe. The gamma rays, which have not been absorbed, contact the radiation detector and ionize a gas contained therein. This ionization produces pulse frequencies which can be related to the fluid interface level in the standpipe. A signal conditioning system then converts these pulse frequencies into useable analog signals which can be used with conventional process controllers. Examples of such signal conditioning systems include, but are not limited to, computer systems and/or microprocessor systems.

When the fluid interface level within the standpipe changes, the absorption of gamma rays by the fluids will also change. This change in absorption of gamma radiation will affect the amount of gas ionized within the radiation detector, thus affecting the pulse frequencies produced therefrom and the analog signals related thereto. These signals can be used to activate level indicators and/or conventional process controllers to indicate and/or maintain a desired fluid interface level within the standpipe. Since, as described above, the standpipe is in fluid communication with the vessel, measuring and/or controlling the interface levels within the vessel will inherently measure and/or control the corresponding interface levels within the vessel.

The process controllers mentioned above may utilize the various modes of control known in the art, such as proportional, proportional-integral, proportional-derivative, or proportional-integral-derivative. In the preferred embodiment, proportional-integral controllers are utilized. It should be noted, however, that any controller capable of accepting two input signals and producing a scaled output signal representative of a comparison of two input signals is within the purview of this invention.

The scaling of an output signal by a controller is well known in control systems art. Essentially, the output of a controller may be scaled to represent any desired factor or variable. An example of this is where a desired level and an actual level are compared by a controller. The controller output could be a signal representative of a desired change in the flow rate of some gas or liquid necessary to make the desired and actual levels equal. On the other hand, the same output could be scaled to represent a percentage or could be scaled to represent a temperature change required to make the desired and actual levels equal. For example, if the controller output can range from 0 to 10 volts, the output signal could then be scaled such that an output signal having a voltage level of 5 volts would correspond to 50 percent, some specified flow rate, or some specified temperature.

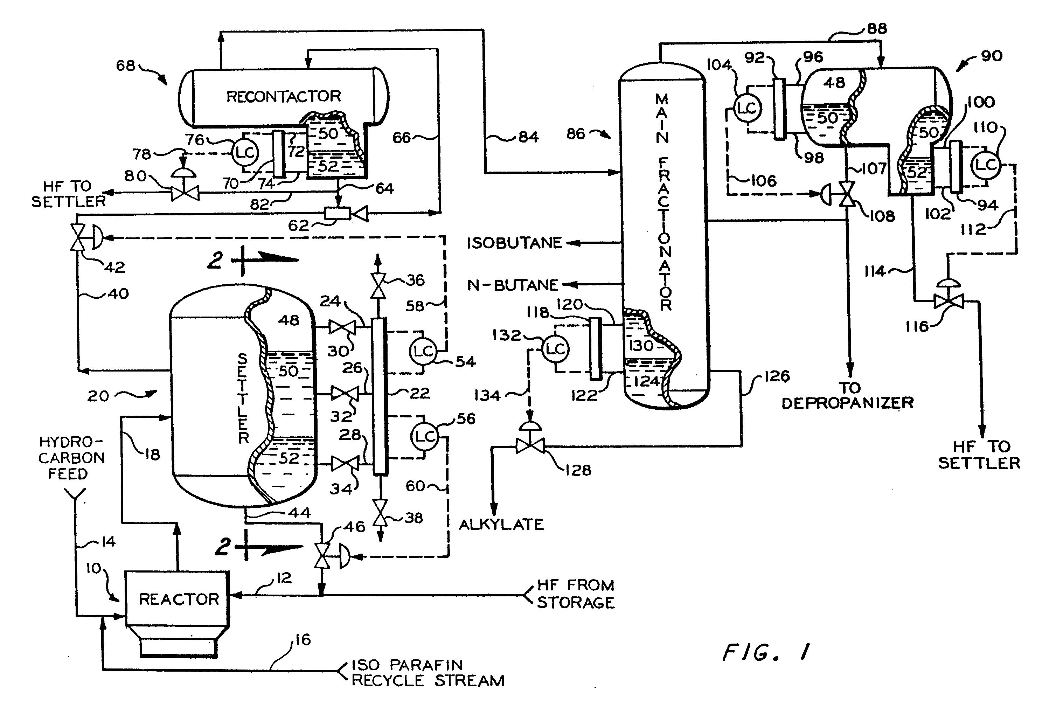

Referring now to the drawings in detail and to **FIGURE 1**, in particular, there is shown an HF Alkylation unit having the level measurement and/or control system of this invention incorporated therein.

Specifically, **FIGURE 1** illustrates an alkylation reactor 10 which has an inlet conduit 12 communicating therewith to supply liquid hydrofluoric acid thereto. A hydrocarbon feedstream, comprising a mixture of hydrocarbons such as isoparaffins and olefins, is introduced into reactor 10 through conduit 14. Optionally, a recycle stream of isoparaffins, such as isobutane, passes through conduit 16 and is mixed with the hydrocarbon feed prior to entering reactor 10.

The alkylation reaction is completed in reactor 10 by intimately contacting the mixed hydrocarbon feed with the hydrofluoric acid. After the alkylation process is completed, the product-containing effluent from reactor 10 is removed through conduit 18 and is fed into settler vessel 20.

In accordance with this invention, settler vessel 20 has associated therewith an externally mounted, generally vertical standpipe 22 which is in fluid communication therewith through conduits 24, 26 and 28 and isolation valves 30, 32 and 34, respectively. At the opposite ends of standpipe 22 are located a vent valve 36 and a drain valve 38. Valves 30, 32 and 34 provide a means for isolating standpipe 22 from settler 20. Once isolated, the nuclear level measurement device can be calibrated without taking settler 20 out of service.

Also associated with settler 20 are withdrawal conduits 40 and 44. Conduit 40 is in fluid communication with the upper end portion of settler vessel 20 and flow control valve 42. Preferably, conduit 40 is located at a position above conduit 18. Conduit 44, on the other hand, is in fluid communication with the lower end portion of settler vessel 20 and flow control valve 46. Preferably, conduit 44 is located below conduit 18.

In operation, the product-containing effluent from alkylation reactor 10 is introduced into settler vessel 20 through conduit 18. Upon being introduced into vessel 20, this effluent separates into three identifiable phases including, vapor hydrocarbon phase 48, liquid hydrocarbon phase 50 and liquid acid phase 52. Liquid hydrocarbon phase 50 generally comprises butanes, propanes and the alkylate product, while liquid acid phase generally comprises hydrofluoric acid and acid-soluble hydrocarbons.

With isolation valves 30, 32 and 34 in their normally open position and valves 36 and 38 in their normally closed position, the product-containing effluent from alkylation reactor 10 also enters standpipe 22 and separates into the same three identifiable phases therein. The levels of the vapor/liquid interface between vapor hydrocarbon phase 48 and liquid hydrocarbon phase 40 and the liquid/liquid interface between liquid hydrocarbon phase 50 and liquid acid phase 52, contained within settler 20, correspond to the same levels within standpipe 22.

Further, in accordance with the present invention, a first level control system 54 is positioned on standpipe 22 at a location between conduits 24 and 26. Level control system 54 is positioned at this location so as to have the ability to detect the vapor/liquid interface within standpipe 22 occurring between vapor hydrocarbon phase 48 and liquid hydrocarbon phase 50.

A second level control system 56 is positioned on standpipe 22 at a location between conduits 26 and 28. Level control system 56 is positioned at this location so as to have the ability to detect the liquid/liquid interface within standpips 22 occurring between liquid hydrocarbon phase 50 and liquid acid phase 52.

Level control system 54 is calibrated such that the vapor/liquid interface between the vapor hydrocarbon phase 48 and liquid hydrocarbon phase 50 within vessel 20 is maintained at a predetermined level. If the actual vapor/liquid interface should deviate from this predetermined level, a signal is transmitted through signal line 58 to flow control valve 42 to accordingly alter the rate at which the liquid hydrocarbons are being withdrawn from vessel 20 through conduit 40.

Level control system 56 is calibrated such that the liquid/liquid interface between the liquid hydrocarbon phase 50 and the liquid acid phase 52 within standpipe 22 is maintained at a predetermined level. If this liquid/liquid interface should deviate from this predetermined level, a signal is transmitted through signal line 60 to flow control valve 46 to accordingly alter the rate at which the liquid acid is being withdrawn from vessel 20 through conduit 44.

If desired, the hydrocarbon effluent from settler vessel 20, which contains some organic fluorides, can optionally pass, via conduit 40, through venturi mixer 62 to convert these organic fluorides, if any, into hydrocarbons and hydrofluoric acid. As the hydrocarbon passes through mixer 62, a low pressure is created which draws hydrofluoric acid from conduit 64 into mixer 62 and intimately mixes the acid with the hydrocarbons passing therethrough. The acid/hydrocarbon mixture then passes through conduit 66 into recontactor 68. Since recontactor 68 is generally run in a full liquid mode, once the acid/hydrocarbon mixture enter recontactor 68 the mixture separates into two identifiable phases including liquid hydrocarbon phase 50 and liquid acid phase 52. The liquid hydrocarbon and liquid acid phases are withdrawn from recontactor 68 through conduits 84 and 64, respectively.

For proper operation of recontactor 68, as little hydrofluoric acid as possible should be withdrawn therefrom through conduit 84. Conversely, as little as possible liquid hydrocarbon should be withdrawn from recontactor 68 through conduit 64. Therefore, recontactor 68 also has associated therewith an externally positioned, generally vertical standpipe 70 which is in fluid communication with recontactor 68 through conduits 72 and 74. Conduit 72 is positioned such that it is at a location above the liquid/liquid interface within recontactor 68 between liquid hydrocarbon phase 50 and liquid acid phase 52. Conduit 74, on the other hand, is positioned such that it is at a location below the liquid/liquid interface within recontactor 68 between liquid hydrocarbon phase 50 and liquid acid phase 52.

It should be noted that the vertical standpipe in fluid communication with recontactor 68 and all those disclosed hereafter include: (1) isolation valves, which are located in the conduits which are in fluid communication with the standpipes with the interior cavity of their respective vessel, (2) at least one vent valve and (3) at least one drain valve as described earlier for standpipe 22. For purposes of simplifying the disclosure and the drawings, these valves will not be illustrated. It is to be understood, however, that, in the preferred embodiment, they do exist.

A third level control system 76 is positioned on standpipe 70. Level control system 76 is positioned between conduits 72 and 74 and is calibrated such that the liquid/liquid interface within standpipe 70, between liquid hydrocarbon acid phase 50 and liquid acid phase 52, is maintained at a predetermined level. If the actual liquid/liquid interface should deviate from this predetermined level, a signal is transmitted, via signal line 78, to a flow control valve 80 to accordingly alter the rate at which liquid acid is withdrawn from recontactor 68 through conduits 64 and 82.

Liquid hydrocarbon phase 50 from recontactor 68 is withdrawn through conduit 84 and introduced into fractionator 86. In operation, the light hydrocarbons, and most of the remaining hydrofluoric acid, if any, are withdrawn from fractionator 86 through overhead conduit 88 into accumulator 90. After the overhead product from fractionator 86 enters accumulator 90 through conduit 88, it separates into three identifiable phases including a vapor hydrocarbon phase 48, a liquid hydrocarbon phase 50 and a liquid acid phase 52. The liquid hydrocarbon and liquid acid phases are withdrawn from accumulator 90 through conduits 107 and 114, respectively.

For proper operation of accumulator 90, as little hydrofluoric acid, as possible, should be withdrawn therefrom through conduit 107. Conversely, as little as possible liquid hydrocarbon should be withdrawn from accumulator 90 through conduit 114. Therefore, accumulator 90 has associated therewith two vertical standpipes 92 and 94. Standpipe 92 is in fluid communication with accumulator 90 through conduits 96 and 98. Standpipe 94, on the other hand, is in fluid communication with accumulator 90 through conduits 100 and 102.

Conduit 96 of standpipe 92 is positioned such that it is located above the vapor/liquid interface within accumulator 90 forming between vapor hydrocarbon phase 48 and liquid hydrocarbon phase 50. Conduit 98 of standpipe 92 is positioned such that it is at a location below the vapor/liquid interface within accumulator 90 forming between vapor hydrocarbon phase 48 and liquid hydrocarbon phase 50. On the other hand, conduit 100 of standpipe 94 is positioned such that it is located above the liquid/liquid interface within accumulator 90 forming between liquid hydrocarbon phase 50 and liquid acid phase 52. Conduit 102 of standpipe 94 is positioned such that it is located below the liquid/liquid interface within accumulator 90 forming between liquid hydrocarbon phase 50 and liquid acid phase 52.

A fourth level control system 104 is position on vertical standpipe 92. Level control system 104 is positioned between conduits 96 and 98 and is calibrated such that the vapor/liquid interface within standpipe 92, between the vapor hydrocarbon phase 48 and the liquid hydrocarbon phase 50, is maintained at or above a predetermined level. If the actual vapor/liquid interface should deviate from this predetermined level, a signal is transmitted, via signal line 106, to flow control valve 108 to accordingly alter the rate at which liquid hydrocarbons are withdrawn from accumulator 90 through conduit 107.

A fifth level control system 110 is positioned on standpipe 94. Level control system 110 is positioned between conduits 100 and 102 and is calibrated such that the liquid/liquid interface within standpipe 94, between the liquid hydrocarbon phase 130 and the liquid hydrocarbon phase 124, is maintained at or below a predetermined level. If the actual liquid/liquid interface should deviate from this predetermined level, a signal is transmitted, via signal line 112, to flow control valve 116 to accordingly alter the rate at which liquid acid is withdrawn from accumulator 90 through conduit 114.

Fractionator 86 is designed such that the bottoms product 124 is the desired liquid phase alkylate product. This alkylate product is withdrawn from fractionator 86 through conduit 126. Also located in the lower portion of fractionator 86 is a vapor hydrocarbon phase 130. Sine it is desirable that the liquid alkylate product be withdrawn from fractionator 86 without also withdrawing, from the same conduit, any vapor hydrocarbons, a vertical standpipe 118 is operatively connected to the lower portion of fractionator 86 via conduits 120 and 122. Conduit means 120 is positioned such that it is at a location above the vapor/liquid interface within fractionator 86 between vapor hydrocarbons phase 130 and vapor hydrocarbons phase 124. Conduit means 122 is positioned such that it is located below the vapor/liquid interface within fractionator 86 between vapor hydrocarbon phase 130 and liquid hydrocarbon phase 124.

A sixth level control system 132 is positioned on standpipe 118. Level control system 132 is positioned between conduits 120 and 122 and is calibrated such that the vapor/liquid interface within standpipe 118, between the vapor hydrocarbon phase 130 and liquid hydrocarbon phase 124 is maintained at a predetermined level. If the actual liquid/liquid interface should deviate from this predetermined level, a signal is transmitted, via signal line 134, to flow control valve 128 to accordingly alter the rate at which the liquid alkylate is withdrawn from fractionator 86 through conduit 126.

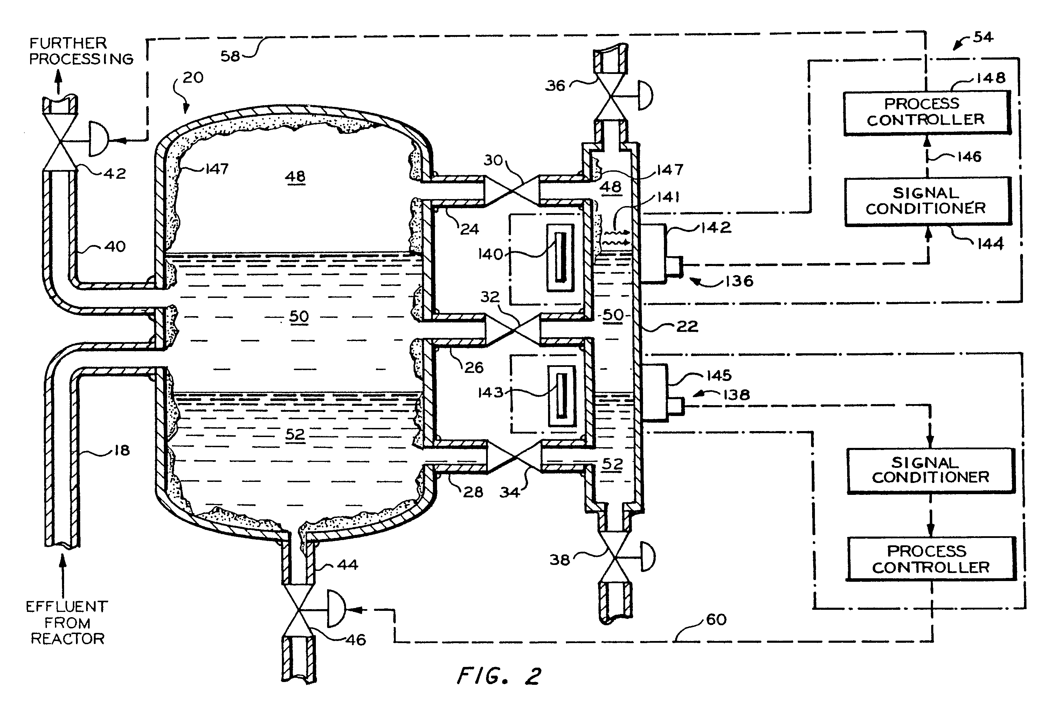

One specific embodiment of the level control system of this invention is illustrated in **FIGURE 2**. As stated earlier, **FIGURE 2** is a cross-sectional view of the hydrocarbon-acid settler vessel 20 of **FIGURE 1** with the level control system of the present invention positioned on a standpipe operatively connected thereto. Settling vessel 20 has associated therewith an inlet conduit 18 through which the product-containing effluent from alkylation reactor 10 is introduced therein. Settler vessel 20 further includes two outlet conduits 40 and 44, wherein conduit 40 is positioned such that it can withdraw liquid hydrocarbons; and, conduit 44 is positioned such that it can withdraw liquid hydrofluoric acid.

In accordance with the present invention, a generally vertical standpipe 22, which can also be viewed as a longitudinal reference zone, is in fluid communication with the internal cavity of vessel 20, which can also be viewed as a phase separation zone, by means of conduits 24, 26, and 28 and isolation valves 30, 32, and 34, respectively. When settler vessel 20 is in operation, isolation valves 30, 32, and 34 are normally opened and 36 and 38 are normally closed. However, if necessary, valves 30, 32, and 34 can be closed to isolate standpipe 22 from vessel 20 and the HF Alkylation unit. While these isolation valves are closed, standpipe 22 can be repaired and/or the nuclear level measurement devices mounted thereon can be calibrated without needing to shutdown the HF Alkylation unit.

Standpipe 22 further includes vent valve 36 and drain valve 38. These valves are generally used when repairing the standpipe and/or calibrating the nuclear level measurement devices mounted thereon.

Standpipe 22 can be made from any material which suitably resists the corrosive tendencies of hydrofluoric acid. Examples of such materials include, but are not limited to, platinum, nickel, gold, silver, copper, low carbon steel (i.e., ASTM 106 Grade B) or nickel alloys such as Hastelloy B, Hastelloy C and Monel. In view of commercial and economic feasibility, the preferred materials from which standpipe 22 can be prepared are low carbon steel or the nickel alloy Monel.

Generally, standpipe 22 will be a length sufficient to span the range over which the interface levels needed to be measured and/or controlled. Moreover, standpipe 22 will generally have an inside diameter sufficient to permit non-restrictive flow of fluids therethrough. Preferably, standpipe 22 will have an inside diameter ranging from about 1 inch to about 12 inches, more preferably, from about 2 inches to about 10 inches, and even more preferably, from about 3 inches to about 8 inches.

Further in accordance with this invention, one or more level control systems can be operatively connected to the aforementioned standpipe. Specifically, as illustrated in **FIGURE 2**, two such level control systems are operatively connected standpipe 22. These level control systems are identified by item number 54 and 56. The level control systems of this invention preferably comprise a nuclear level measurment device, a signal conditioner, and a process controller and/or a level indicating device.

In a preferred embodiment of this invention, the nuclear level measurment devices 136 and 138 comprise strip-type sources of radioactive material 140 and 143 disposed on one side of standpipe 22 and gas-containing radiation detector cells 142 and 145 disposed on the opposite side thereof. Gamma radiation is emitted from the radiation source 140 as collimated beam 141 through the walls of standpipe 22, scale formations 147, if any, and the material whose level is to be detected. When the gamma rays contact the gas within radiation detection cell 142, the gas is ionized. This ionization establishes a first signal respective of the actual level of the vapor/liquid interface within standpipe 22. The first signal can be used, in one instance, to actuate a level indicating device (not shown).

In a preferred embodiment, a second signal (not shown) is established by signal conditioner 144 representative of the desired level of the vapor/liquid interface within standpipe 22. Signal conditioner 144 then established a third signal 146 which represents the difference between the first signal and the second signal.

Signal 146 can be scaled to be used in conjunction with a level recording instrument or with a process controller to actuate suitable apparatus such as electrically or pneumatically controlled valves for maintaining the vapor/liquid interface and/or liquid/liquid interface within standpipe 22 and vessel 20 at their respective predetermined levels. If desired, signal 146 can perform two or more of these functions simultaneously. For example, it is possible that signal 146 can be used to control the vapor/liquid interface level by actuating process controller 148 to manipulate flow control valve 42 and simultaneously provide a visual indication of the interface level by actuating a recording instrument (not shown).

Any suitable nuclear level detecting device can be used for practicing the embodiments of this invention. Examples of manufacturers which can provide such suitable nuclear measurment devices include, Texas Nuclear, of Austin, Texas; Ohmart Corporation of Cincinnati, Ohio; and Kay-Ray Inc. of Arlington Heights, Illinois. Nuclear level measurment devices specifically suitable for such an application are Texas Nuclear's CND (E-Zcal) Continuous Level Controllers, model numbers 5195 and 5196.

In a preferred embodiment, the strip-type radiation source 140 is disposed vertically adjacent an external wall of standpipe 22, while radiation detection cell 142 is disposed vertically adjacent to the external wall of standpipe 22 opposite of radiation source 140. The positioning of radiation source 140 and radiation detector 142 is such that the radiation emitted must pass through the at least part of the fluid within standpipe 22 before impinging upon radiation detection cell. Moreover, it should be noted that radiation detection cell 142 should be positioned such that it spans the range of interface levels to be measured and/or controlled thereby.

Prior to being used as an integral part of the inventive level control system, the nuclear level measurment device must be calibrated. This calibration process can be accomplished by many different means, one such method of calibrating measurement devices 136 and 138 is by isolating standpipe 22 from vessel 20 by first closing isolation valves 30, 32 and 34. After the isolation valves have been closed, drain valve 38 and vent valve 36 are opened to remove any fluid contained within standpipe 22. After all fluid has been removed, drain valve 38 is closed and standpipe 22 is filled with the various fluids whose levels are to be measured and controlled by selectively opening the appropriate isolation valve. After the desired process fluid has been introduced into standpipe 22, the nuclear measurement devices are then calibrated accordingly so as to maintain the vapor/liquid interface and/or liquid/liquid interface within a desired range or at a desired level. After calibrating nuclear measurment devices 136 and 138, standpipe 22 is drained, vent valve 36 and drain valve 38 are closed and isolation valves 30, 32 and 34 are reopened.

As can be seen from the above disclosure, the inventive level control system circumvents many of the calibration problems associated with level controlling systems wherein the nuclear level measurement device is operatively connected to the vessel, as opposed to an externally positioned onto a standpipe. For example, since the standpipe of this invention has a much smaller volume than that of the vessel, the amount of fluids necessary during the calibration process of the nuclear measurement devices is very small when compared to that necessary for calibrating a vessel-mounted nuclear measurment device. Moreover, since the standpipe can easily be repaired and/or replaced without shutting down the HF Alkylation unit, it need not be constructed so as to withstand the years of service required from an alkylation vessel. Therefore, the standpipe can be constructed of materials having wall thicknesses which are thinner than those generally used in the fabrication of alkylation vessels. Since the wall thickness of the standpipe is generally thinner than that of the alkylation vessel, and since the diameter of the standpipe is substantially less than that of the alkylation vessel, it follows that the strength of the radiation source necessary to practice the embodiments of this invention will also be substantially less than that needed if the nuclear measurment device was operatively connected to the vessel as opposed to the standpipe.

Another problem circumvented by the inventive level controlling system disclosed herein is that encountered when attempting to measure and/or control the interface levels within an irregularly shaped vessel or within a vessel containing baffles, trays, and/or pipes therein. Specifically, regardless of the shape or fixtures within the vessel, the interface levels therein will correspond with those within the vertical standpipe. Thus, by measuring and/or controlling the interface levels within the vertical standpipe, the corresponding interface levels within the irregularly shaped vessel will also be controlled.

In the operation of the specific embodiment illustrated in **FIGURE 2**, wherein the novel level controlling system is used to measure and control the interface levels occurring within an HF alkylation process, the product-containing effluent from alkylation reactor 10 is introduced into vessel 20 through conduit 18. As stated earlier, when the effluent from the alkylation reactor enters settler vessel 20, the effluent separates into three identifiable phases. These phases are a vapor hydrocarbon phase 48, liquid hydrocarbon phase 50 and liquid hydrofluoric acid phase 52. In proper operation of settler vessel 20, as little as possible liquid acid and/or vapor hydrocarbon should be withdrawn therefrom through conduit 40. Therefore, the liquid hydrocarbon level within vessel 20 should not fall below the opening of vessel 20 into conduit 40. Moreover, the liquid acid phase should not rise above the opening of vessel 20 into conduit 40. Preferably, however, the liquid acid phase not rise above the opening of vessel 20 into conduit 18. In order to maintain the vapor/liquid interface between vapor hydrocarbon phase 48 and liquid hydrocarbon phase 50 at a level above the opening of vessel 20 into conduit 40, nuclear level measurement device 136 is attached to standpipe 22 at a location such that the lower end of radiation strip source 140 is at or above the fluid communication opening of vessel 20 into conduit 40. Moreover, in order to maintain the liquid/liquid interface between liquid hydrocarbon phase 50 and liquid acid phase 52 below the fluid communication opening of vessel 20 into conduit 40, and preferably below the fluid communication opening of vessel 20 into conduit 18, nuclear level measurement device 138 is attached to standpipe 22 such that the upper end of radiation strip source 143 is at or below the fluid communication opening between the internal cavity of vessel 20 and conduit 18.

It is evident from the foregoing that various modifications can be made to the embodiments of this invention without departing from the spirit and scope thereof which will be apparent to those skilled in the art. Having thus described the invention, it is claimed as follows.

## Claims

1. An apparatus comprising:
a vessel for containing fluids, said vessel having at least one fluid inlet conduit and at least one fluid outlet conduit;
a standpipe having an upper end portion, a medial portion and a lower end portion, said standpipe being generally vertically positioned external to said vessel;
a first conduit means opening at one end into said vessel and opening at the other end into said lower end portion of said standpipe for passing fluids from said vessel into said standpipe;
a second conduit means opening at one end into said vessel and opening at the other end into said upper end portion of said standpipe for passing fluids from said vessel into said standpipe; and
a level control system comprising a nuclear level measuring device which includes a radiation emission source adjacent an external wall of said standpipe at a location below said first conduit means and above said second conduit means and a radiation detection means for detecting radiation emitted by said radiation source and transmitting a signal relating thereto, said radiation detection means adjacent to the external wall of said standpipe opposite of that which said radiation emission source is adjacent, such that the radiation emitted from said radiation emission source passes through the wall of said standpipe directly adjacent said radiation emission source, a cavity formed by the internal walls of said standpipe, and the wall of said standpipe directly adjacent said radiation detection means prior to contacting said radiation detection means.

2. An apparatus in accordance with claim 1 wherein said vessel is at least one of the vessels suitable for use in an alkylation process of hydrocarbons with a hydrofluoric acid catalyst.

3. An apparatus in accordance with claim 2 wherein said vessel is at least one of a hydrofluoric acid settler, an acid recontactor, a fractionator or an accumulator.

4. An apparatus in accordance with claim 1 wherein said standpipe is constructed of a material selected from the group consisting of platinum, nickel, gold, silver, copper, low carbon steel, Hastelloy B, Hastelloy C, and Monel.

5. An apparatus in accordance with claim 4 wherein said standpipe is constructed from a material selected from the group consisting of low carbon steel and Monel.

6. An apparatus in accordance with claim 1 wherein said standpipe has a cross sectional area less than that of said said vessel.

7. An apparatus in accordance with claim 1 wherein said standpipe includes a vent valve in fluid communication with with the upper end portion of said standpipe and a drain valve in fluid communication with the lower end portion of said standpipe.

8. An apparatus in accordance with claim 1 wherein said standpipe has an inside diameter ranging from about 1 inch to about 12 inches.

9. An apparatus in accordance with claim 8 wherein said standpipe has an inside diameter ranging from about 2 inches to about 10 inches.

10. An apparatus in accordance with claim 9 wherein said standpipe has an inside diameter ranging from about 3 inches to about 8 inches.

11. An apparatus in accordance with claim 1 further including:
a first isolating valve means operatively connected to said first conduit means for enabling the control of said fluid flowing between said vessel and said standpipe through said first conduit means; and
a second isolating valve means operatively connected to said second conduit means for enabling the control of said fluid flowing between said vessel and said standpipe through said second conduit means.

12. An apparatus in accordance with claim 1 wherein said level control system further includes a signal conditioning system.

13. An apparatus in accordance with claim 12 wherein said signal conditioning system at least one of a computer or a microprocessor.

14. An apparatus in accordance with claim 12 wherein said level control system further includes a process controller.

15. An apparatus in accordance with claim 14 further including a flow control valve in fluid communication with said at least one fluid outlet means, said flow control valve being operatively connected to said process controller.

16. An apparatus in accordance with claim 12 wherein said level control system further includes a level indicating device.

17. An apparatus in accordance with claim 14 wherein said level control system further includes a level indicating device.

18. An apparatus in accordance with claim 1 wherein said radiation source of said nuclear level measuring device comprises a strip-type radiation source.

19. An apparatus in accordance with claim 1 wherein said radiation source is selected from the group consisting of cesium-137 and cobalt-60.

20. An apparatus in accordance with claim 18 wherein said radiation source is selected from the group consisting of cesium-137 and cobalt-60.

21. An apparatus in accordance with claim 1 further comprising a third conduit means opening at one end into said vessel and opening at the other end into said medial portion of said standpipe.

22. An apparatus in accordance with claim 21 further including:
a first isolating valve means operatively connected to said first conduit means for enabling the control of said fluid flowing between said vessel and said standpipe through said first conduit means;
a second isolating valve means operatively connected to said second conduit means for enabling the control of said fluid flowing between said vessel and said standpipe through said second conduit means; and
a third isolating valve means operatively connected to said third conduit means for enabling the control of said fluid flowing between said vessel and said standpipe through said third conduit means.

23. An apparatus in accordance with claim 21 wherein said nuclear level measuring device is positioned on said standpipe at a location above said first conduit means and below said third conduit means.

24. An apparatus in accordance with claim 21 wherein said nuclear level measuring device is positioned on said standpipe at a location below said second conduit means and above said third conduit means.

25. An apparatus in accordance with claim 23 further including a second nuclear level measuring device positioned on said standpipe at a location above said first conduit means and below said third conduit means.

26. An apparatus in accordance with claim 25 further including:
a first isolating valve means operatively connected to said first conduit means for enabling the control of said fluid flowing between said vessel and said standpipe through said first conduit means;
a second isolating valve means operatively connected to said second conduit means for enabling the control of said fluid flowing between said vessel and said standpipe through said second conduit means; and
a third isolating valve means operatively connected to said third conduit means for enabling the control of said fluid flowing between said vessel and said standpipe through said third conduit means.

27. An apparatus in accordance with claim 26 wherein said standpipe includes a vent valve in fluid communication with with the upper end portion of said standpipe and a drain valve in fluid communication with the lower end portion of said standpipe.

28. A process for determining the level of a primary interface of two fluids within a phase separation zone, said primary interface resulting between a first fluid and a second fluid, wherein said first fluid and said second fluid are insoluble in the other and said first fluid has a specific gravity greater than that of said second fluid, wherein a substantial portion of said first fluid is removed from said phase separation zone through a first outlet stream positioned below the level of said primary interface, comprising the steps of:
passing a portion of said first fluid from said phase separation zone into a longitudinal reference zone through a first conduit means positioned below the level of said primary interface and passing a portion of said second fluid from said phase separation zone into said longitudinal reference zone through a second conduit means positioned above the level of said primary interface, such that a secondary interface between said first fluid and said second fluid is formed in said longitudinal reference zone, said secondary interface corresponding with said primary interface;
passing nuclear radiation, emitted by a radiation source, through said longitudinal reference zone in a direction generally perpendicular to the longitudinal axis of said longitudinal reference zone in a manner such that said nuclear radiation contacts said first fluid and said second fluid;
detecting said nuclear radiation passing through said longitudinal reference zone with a radiation detection device, wherein said radiation detection device spans said secondary interface within said longitudinal reference zone;
establishing a first signal responsive to said nuclear radiation detected by said radiation detection device, wherein said first signal is scaled so as to be representative of the actual level of said secondary interface within said longitudinal reference zone and corresponding said primary interface level within said phase separation zone; and
actuating a level indicating device in response to said scaled first signal.

29. A process in accordance with claim 28 wherein said first fluid comprises a liquid acid and said second fluid comprises acid-insoluable liquid hydrocarbons.

30. A process in accordance with claim 29 wherein said acid comprises hydrofluoric acid.

31. A process in accordance with claim 29 wherein said acid-insoluble hydrocarbons comprises a mixture of butanes and propanes.

32. A process in accordance with claim 31 wherein said acid comprises hydrofluoric acid.

33. A process in accordance with claim 28 wherein said first fluid comprises a vapor and said second fluid comprises a liquid.

34. A process in accordance with claim 33 wherein said vapor comprises vaporized hydrocarbons.

35. A process in accordance with claim 33 wherein said liquid comprises liquid hydrocarbons.

36. A process in accordance with claim 35 wherein said vapor comprises vaporized hydrocarbons.

37. A process in accordance with claim 36 wherein said vaporized hydrocarbons comprise propanes and said liquid hydrocarbons comprise butanes.

38. A process in accordance with claim 28 wherein said radiation source is a strip-type radiation source.

39. A process in accordance with claim 38 wherein said strip-type radiation source is selected from the group consisting of cesium-137 and cobalt-60.

40. A process for controlling the level of a primary interface of two fluids within a phase separation zone, said primary interface resulting between a first fluid and a second fluid, wherein said first fluid and said second fluid are insoluble in the other and said first fluid has a specific gravity greater than that of said second fluid, wherein a substantial portion of said first fluid is removed from said phase separation zone through a first outlet stream positioned below the level of said primary interface, comprising the steps of:
passing a portion of said first fluid from said phase separation zone into a longitudinal reference zone through a first conduit means positioned below the level of said primary interface and passing a portion of said second fluid from said phase separation zone into said longitudinal reference zone through a second conduit means positioned above the level of said primary interface, such that a secondary interface between said first fluid and said second fluid is formed in said longitudinal reference zone, said secondary interface corresponding with said primary interface;

passing nuclear radiation, emitted by a radiation source, through said longitudinal reference zone in a direction generally perpendicular to the longitudinal axis of said longitudinal reference zone in a manner such that said nuclear radiation contacts said first fluid and said second fluid;

detecting said nuclear radiation passing through said longitudinal reference zone with a radiation detection device, wherein said radiation detection device spans said secondary interface within said longitudinal reference zone;

establishing a first signal responsive to said nuclear radiation detected by said radiation detection device representative of the actual level of said secondary interface within said longitudinal reference zone and corresponding said primary interface level within said phase separation zone;

establishing a second signal respresentative of the desired level of said secondary interface within said longitudinal reference zone which corresponds to a desired level of said primary interface within said phase separation zone;

comparing said first signal and said second signal and establish a third signal which is represents the difference between said first signal and said second signal; and

manipulating the flow rate of said first fluid from said phase separation zone through said first outlet stream to maintain the actual level of said primary interface and corresponding said secondary interface substantially equal to the desired level represented by said second signal.

41. A process in accordance with claim 40 wherein said step of manipulating the flow rate of said first fluid from said phase separation zone through said first outlet stream comprises:

scaling said third signal to be representative of the position of a control valve, operably located so as to control the flow rate of said first fluid, required to maintain the actual level of said primary interface and corresponding said secondary interface substantially equal to the desired level represented by said second signal; and

manipulating the position of said control valve in response to said scaled third signal.

42. A process in accordance with claim 40 further comprising:

establishing a fourth signal responsive to said nuclear radiation detected by said radiation detection device, wherein said fourth signal is scaled so as to be representative of the actual level of said secondary interface within said longitudinal reference zone and corresponding said primary interface level within said phase separation zone; and

actuating a level indicating device in response to said scaled fourth signal.

FIG. 1

FIG. 2

FURTHER
PROCESSING

EFFLUENT
FROM
REACTOR

PROCESS
CONTROLLER

SIGNAL
CONDITIONER

SIGNAL
CONDITIONER

PROCESS
CONTROLLER

EP 0 330 123 A2